# EUROPEAN PATENT SPECIFICATION

(11) **EP 1 711 070 B1**
(45) Date of publication and mention of the grant of the patent: **09.12.2015**
(21) Application number: 05701218.9
(22) Date of filing: 27.01.2005
(51) Int. Cl.: A23L 1/30, A61K 31/205

(54) **NUTRITIONAL COMPOSIITION FOR IMPROVING SKIN CONDITION AND PREVENTING SKIN DISEASES**
NÄHRZUSAMMENSETZUNG ZUR VERBESSERUNG DES HAUTZUSTANDES UND ZUR VORBEUGUNG GEGEN HAUTKRANKHEITEN
COMPOSITION NUTRITIONNELLE CONÇUE POUR AMELIORER L'ETAT DE LA PEAU ET PREVENIR DES MALADIES DE LA PEAU

(30) Priority: 28.01.2004 EP 04001843
(43) Date of publication of application: 18.10.2006
(73) Proprietor: Nestec S.A., 1800 Vevey (CH)
(72) Inventor: PRIDMORE-MERTEN, Sylvie, 1000 Lausanne 26 (CH)
(74) Representative: Rosolen-Delarue, Katell
(86) International application number: PCT/EP2005/000808
(87) International publication number: WO 2005/074719

(56) References cited:
- WO-A-02/11717
- RU-C1- 2 187 229
- US-A1- 2002 077 349
- US-A1- 2003 060 503
- US-B1- 6 503 506

## Description

The present invention pertains to the use of an ingestable composition containing L-carnitine and at least one component having an anti-oxidative activity for stimulating the lipid metabolism in the skin of an individual, which results in an improvement of the general condition of the skin, in particular dry, sensible or reactive skin, and in prevention of the occurrence of skin diseases, such as dermatitis. In particular the present invention relates to the use of such a composition for the preparation of a pharmaceutical composition or of a food, in particular a functional food.

The skin is the largest organ of the body and serves as a protective shield against environmental influences, such as temperature or physical bearings. The skin also assists in regulating the body temperature, stores water and fat and prevents entry of pathogens into the body. Daily life in our industrial society has a detrimental impact on the skin in that environmental factors, such as air pollution, or stress in profession affect the skin's condition, which in the long run result in a deterioration of the skin's status and eventually performance.

In general, for preventing deterioration or even improving the condition and/or performance of a skin's two approaches are known. The first approach resides in applying topical formulations directly on the skin, such as creams, ointments or lotions, which may prove effective under certain circumstances. These formulations do, however, suffer from the shortcomings that the symptoms instead of underlying causes of a deterioration or a disease in the skin are treated.

A second approach for improving the skin's condition involves administration of ingestible compositions designed to act from within the body. Several such compositions have been proposed in the art, which, however, often contain rare and expensive starting materials of plant or animal origin. The Jackson Laboratory (USA) has reported that a reduction of the dietary fat content may lead to a reduction of the incidence of specific skin diseases, such as dermatitis in certain strains of mice. The reduction of fat content in the diet did, however, only postpone skin disease and did not suppress ulcerative dermatitis. In addition, most of these compositions focus on individuals of a specific age, e.g. either young individuals or elderly persons, or only women, and are not suited for long-term consumption due to the ingredients they contain.

In view of the shortcomings of the prior art, the problem of the present invention resides in providing a nutritional composition, which may be used for a long-term consumption, and provides relief to all individuals irrespective of their age and/or gender by improving the skin condition and/or by preventing skin diseases.

This problem has been solved by using an ingestible composition containing a mixture of L-carnitine and at least one compound having an anti-oxidative activity, for stimulating the lipid metabolism in the skin of an individual.

During the intensive research work directed to the consequences of varying the caloric supply to individuals of different age, the present inventors surprisingly noted that among the several diets administered, one diet surprisingly stimulated the lipid metabolism and thereby provided a protective effect on the condition of the skin (e.g. dry, sensible or reactive skin) while also assisting in preventing skin diseases, in particular ulcerative dermatitis. Based on this findings, subsequent studies have been carried out, which led to the identification of the essential ingredients, which yield the effect observed.

Even more surprising, this beneficial effect could be observed when a diet relatively rich in fat has been given, which is essentially contrary to the general understanding in the art in that a significant improvement of the skin's condition may be obtained only by reducing the content of fat in an individual's diet. This trait seems to be rather favourable, in that the normal diet in Western societies contains a high amount of fat.

The lipid metabolism stimulating activity as observed when using the ingestable composition may go along with an increase of lipids in sebum.

These effects will lead to an overall improvement of the skin's condition and prevents the onset/incidence of a skin disease, such as dermatitis, in particular ulcerative dermatitis, ulcers associated with diabetes, circulation disturbances, mechanical trauma, physical, chemical or microbial noxae or eczema. The ingestable composition is also intended for reduction of itching. In addition, when using the present composition an improvement of the skin condition of individuals may be observed, that have a decreased lipid secretion in sebum, one of several causes of a condition often designated as "dry skin" or "sensible skin".

The term "ingestable composition", as used in the present application comprises any food-acceptable composition.

The term "improving skin condition and preventing skin diseases" comprises not only the improvement of skin conditions and a prevention of skin diseases, but also a balancing of the processes occurring in the dermis. A nutritional composition according to the present invention stimulates the lipid metabolism an in particular increases lipid secretion in sebum, and helps thereby to produce a protective sebum layer on the skin. As well known sebum is a complex mixture of fats, cells, free acids, which mixture is secreted by sebaceous glands and protects the skin from external influences. Without wishing to be bound to any theory, it may be therefore assumed that for an increasing of the lipid secretion in sebum various different and interacting metabolic processes in the body have to be influenced.

The term "L-carnitine" as used in the present application comprises both, the naturally occurring amino acid L-carnitine, as well as acyl-derivatives of L-camitine, such as e.g. the acetyl-derivative of L-carnitine or the propionyl L-carnitine. Said compounds may be isolated from natural sources, or may be synthesized chemically. The preferred ingredient is the naturally occurring amino acid L-carnitine.

The term "component having an anti-oxidative activity" or "anti-oxidative component" comprises any food-acceptable components having an anti-oxidative activity, such as vitamin C, vitamin E (tocopherols and tocotrienols) and derivatives thereof, such as e.g. vitamin E acetate, carotenoids (carotenes, lycopene, lutein, zeaxanthine), ubiquinones (e.g. CoQ10), tea catechins (e.g. epigallocatechin gallate), coffee extracts containing polyphenols and/or diterpenes (e.g. kawheol and cafestol), ginkgo biloba extracts, grape or grape seed extracts rich in proanthocyanidins, spice extracts (e.g. rosemary), soy extracts containing isoflavones, phytoestrogens, compounds that upregulate cell antioxidant defense (e.g. ursodeoxycholic acid for increased glutathione S-transferase, ursolic acid for increased catalase, ginseng and gingenosides for increased superoxide dismutase and natural sources thereof, such as herbal medicines), compounds being a source of thiols, such as e.g. lipoic acid, cysteine, cystine, methionine, S-adenosyl-methionine, taurine, glutathione, natural sources thereof, or compounds up-regulating their in vivo biosynthesis, as well as mixtures of anti-oxidative components, such as e.g. mixtures of the above-mentioned anti-oxidative components.

The amount of L-carnitine to be administered daily to an individual is at least 1 mg per kg of body weight per day, and is preferably in the range of from 1 mg to 1 g per kg of body weight per day, and more preferably in the range of from 5 mg to 250 mg per kg of body weight per day. Especially for humans, L-carnitine may be chosen in an amount of 7 to 70 mg per kg of body weight per day, in particular of 29 to 31 mg per kg of body weight per day. For pets, such as dogs and cats, L-carnitine may be chosen in an amount of 21 to 210 mg per kg of body weight per day, in particular of 19 to 21 mg per kg of body weight per day. In case of L-carnitine derivatives, the corresponding amounts are varied based on the molecular weight of the compound.

A complete nutritional composition of a human or mammalian food in essentially dry form may comprise for example up to 1800 to 2200 ppm (parts per million) of L-carnitine or a derivative, thereof, more preferably up to 750 ppm of L-carnitine or derivative thereof, most preferably 510 to 750 ppm of L-carnitine or derivative thereof.

Nutritional compositions comprising an amount of anti-oxidative component(s), in an amount of for example at least 0.025 mg per kg of body weight per day, preferably from 0.025 mg to 250 mg per kg of body weight per day, provided good results.

An example for a combination of anti-oxidative components is e.g. a combination of vitamin E (or derivatives thereof) with at least one additional anti-oxidative component, preferably an anti-oxidative component having a higher water-solubility as compared to vitamin E, such as a combination of vitamin E and at least one, preferably two or three of the antioxidative components selected from the group consisting of vitamin C or derivatives thereof, grape seed extract, and cysteine.

An example for vitamin E or a derivative thereof for use in a nutritional composition according to the present invention is e.g. alpha-tocopherylacetate, in particular in the (all rac)-configuration, which is commercially available. Vitamin E or said derivative may be present in an amount corresponding 2 to 6-times, preferably 4,5 to 5.5-times the AFFCO recommendation as a daily dosage.

Without wishing to be bound to any theory, sebum appears to be the major physiological route for vitamin E secretion in human facial skin and it is assumed that Vitamin E supplementation may act as a protection against lipid oxidation, in particular in the sebum.

Vitamin C may be chosen in an amount of 1 to 4 times the amount of vitamin E, preferably 1.5 to 3 times the amount of vitamin E, more preferably 1.7 to 2.3 times the amount of vitamin E, most preferably 2 times the amount of vitamin E. An example for vitamin C or a derivative thereof for use in a nutritional composition according to the present invention is e.g. stabilized vitamin C commercially available from Roche.

An example for grape or grape seed extracts rich in proanthocyanidins for use in a nutritional composition according to the present invention are Grape seed proanthocyanidin extracts (GSPE) comprising monomers and polymers of catechins and tannins, which are commercially available e.g. under the name Grapemax (Burgundy Botanical extract) or Gravinol (Kikkoman). Grapemax contains e.g. 25 % catechins, 45 % oligomers (2 to 6mers) and 35 % tannins. GSPE may be included for example in an amount of 0.02 to 100 mg per kg of body weight, more preferably 2.5 to 40 mg per kg of body weight as a daily dosage in a nutritional composition according to the present invention. Especially for humans 2.5 to 10 mg of GSPE per kg of body weight, and especially for pet animals, e.g. cats and dogs, 7.5 to 30 mg per kg of body weight could be used as a daily dosage.

Nutritional compositions wherein the mole ratio of L-carnitine and/or acyl-derivative(s) thereof with respect to anti-oxidative component(s), i.e. the amount of L-carnitine and/or derivative(s) thereof in [mol]) / amount of anti-oxidative component(s) in [mol]) is in the range of 2:1 to 100:1, more preferably in the range of between 4:1 and 50:1, most preferably in the range 5:1 and 10:1 provided in particular good properties.

The term "source of fat" as used in the present application comprises any food-acceptable fat(s) and/or oil(s) irrespective of their consistency at room temperature, i.e. irrespective whether said "source of fat" is present in essentially fluid form, such as e.g. soy oil, or in essentially solid from such as e.g. hallow. Thus, in the context of the present invention the terms "fat(s)" and "oil(s)" may be used interchangeably. The term "unsaturated fatty acids" as used herein comprises monounsaturated fatty acids, i.e. fatty acids having one C-C double-bond, as well as fatty acids having more than one C-C double-bond:

The nutritional compositions according to the present invention may comprise fats of animal and/or vegetable origin. For example, vegetable oils such as corn oil, sunflower oil, safflower oil, rapeseed oil, soy bean oil, olive oil, borage oil, blackcurrent seed oil, peanut oil, rice bran oil, and animal oils, such as tallow, in particular beef tallow, and fish oil may be used. More preferably, the source of fat may comprise unsaturated fatty acids, or is enriched with a fat comprising unsaturated fatty acids. The source of fat (either in its natural form or after an enrichment) may comprise at least 30 wt.-% of unsaturated fatty acids (including unsaturated fatty acids bound as glycerin esters, free unsaturated fatty acids, or unsaturated fatty acids bound in any other form) each on basis of total content of fatty acids present in said source of fat. The source of fat may also comprise essential fatty acids (linoleic and alpha-linoleic acid) and polyunsaturated fatty acids, such as gamma linoleic acid, arachidonic acid, eicosapentanoic acid, and docosahexanoic acid.

Fish oils are a suitable source of eicosapentanoic acids and docosahexanoic acid, while borage oil, blackcurrent seed oil and evening primrose oil are suitable sources of gamma linoleic acid. Rapeseed oil, soybean oil, linseed oil and walnut oil are suitable sources of alpha-linoleic acid. Safflower oils, sunflower oils, corn oils and soybean oils are suitable sources of linoleic acid. Olive oil, rapeseed oil (canola) high oleic sunflower and safflower, peanut oil, rice bran oil are suitable sources of monounsaturated fatty acids.

Preferred fats which may form the basis for a source of fat and may be used for the preparation of a nutritional composition according to the present invention are hallow, in particular beef hallow, and soybean oil. In particular, said source of fat may also comprise fatty acids belonging to the family of n-3 polyunsaturated fatty acids, and in particular alpha-linolenic acid. Without wishing to be bound to any theory, for example alpha-linolenic acid is deemed to be a precursor substance in the mammalian metabolism for the formation of eicospentaenoic acid, prostaglandins of the 3-series and leukotrienes of the 5-series and is supposed to provide beneficial effects on the human body and on the human skin.

In a preferred embodiment, if the source of fat used for the preparation of a nutritional composition according to the present invention is soybean oil, then the composition may comprise at least 1 wt.-%, and up to about 7 wt.-% of fatty acids in n-3 form, for example.

Advantageously, said n-3 fatty acids and are present in a weight ratio of (n-6 fatty acids) to (n-3 fatty acids) of from 3:1 to 10:1, more preferably of from 4:1 to 8:1. If necessary, different animal or vegetable oils may be combined in order to obtain a desired composition of the fat source, for example fats rich in n-3 fatty acids may be added to a fat or a composition of fats having a lower content of n-3 fatty acids.

Depending on the formulation intended for the nutritional composition, the fat content may vary. In case of a nutritional formula intended to provide a complete meal or snack or in case of a nutritional supplement to be consumed with a meal having a relatively low fat content, the nutritional composition may comprise up to 40 wt.-%, preferably of from 6 to 25 wt.-%, more preferably of from 10 to 12 wt.-% of a source of fat on basis of the total weight of the nutritional composition. In case of a nutritional supplement intended for consumption with nutrition having a relatively high fat content, the nutritional composition may comprise said source of fat in an amount of at least 0.5 wt.-% on basis of the total weight of the nutritional composition.

The nutritional composition according to the present invention may be further supplemented with a food-acceptable carbohydrate source and/or a source of protein, amino acids, and/or peptides. For example, the carbohydrate source may be flour, grain or starches of e.g. rice, barley, sorghum, millet, oat, corn. Sugars, in particular di- and oligosaccharides, such as e.g. sucrose, glucose and corn syrups may also be used. Additionally also appropriate fibers may be added. The amount of carbohydrate provided by the carbohydrate source may be selected as desired. For example, the food may contain up to about 65 wt.-% of carbohydrate, more preferably 45 to 60 % wt.-% of carbohydrate, on the basis of the total weight of the nutritional composition.

Suitable protein sources for the nutritional composition according to the present invention may be selected from any suitable animal or vegetable protein source, for example muscular or skeletal meat, meat and bone meal, poultry meal, fish meal, milk proteins, corn gluten, wheat gluten, soy flour, soy protein concentrates, soy protein isolates, egg proteins, dietary proteins, such as whey, casein, gluten, and the like. The amount of protein, peptides, and amino acids (without considering the amount of L-carnitine) may be selected as desired. For example, the nutritional composition may contain from about 12 wt.-% to about 70 wt.-% of protein on the basis of the dry weight of the total composition.

The choice of the carbohydrate, protein, peptide, amino acid sources may be selected based upon nutritional needs of its consumer, palatability considerations, and the type of product produced. Further, various other ingredients, for example, salts, spices, seasonings, vitamins, minerals, flavoring agents, gums, prebiotics and probiotic micro-organisms may also be incorporated into the nutritional composition as desired.

The prebiotics may be provided in any suitable form. For example, the prebiotic may be provided in the form of plant material, which contains the prebiotic. Suitable plant materials are e.g. asparagus, artichokes, onions, wheat, yacon or chicory, or residues of these plant materials. Alternatively, the prebiotic may be provided as an inulin extract or its hydrolysis products commonly known as fructo-oligosaccharides, galacto-oligosaccarides, xylo-oligosaccharides or oligo-derivatives of starch. Extracts from chicory are particularly suitable. The maximum level of prebiotic in the nutritional composition is preferably about 20% by weight, especially about 10% by weight. For example, the prebiotic may comprise about 0.1% to about 5% by weight of the nutritional composition. For nutritional composition which use chicory as the prebiotic, the chicory may be included to comprise about 0.5 wt.-% to about 10 wt.-% of the nutritional composition, more preferably about 1 wt.-% to about 5 wt.-%, each on basis of the dry weight of the total composition.

The probiotic micro-organism may be selected from one or more microorganisms suitable for human consumption and/or for a consumption by mammals and which is able to improve the microbial balance in the intestine. Examples of suitable probiotic micro-organisms include yeast such as *Saccharomyces, Debaromyces, Candida, Pichia* and *Torulopsis,* moulds such as *Aspergillus, Rhizopus, Mucor,* and *Penicillium* and *Torulopsis* and bacteria such as the genera *Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus*, *Oenococcus* and *Lactobacillus.* Specific examples of suitable probiotic micro-organisms are: *Saccharomyces cereviseae, Bacillus coagulans, Bacillus licheniformis, Bacillus subtilis, .Bifidobacterium bifidum, Bifidobacterium infantis, Bifidobacterium longum, Enterococcus faecium, Enterococcus faecalis, Lactobacillus acidophilus, Lactobacillus alimentarius, Lactobacillus casei* subsp. *casei, Lactobacillus casei Shirota, Lactobacillus curvatus, Lactobacillus delbruckii* subsp. *lactis, Lactobacillus farciminus, Lactobacillus gasseri, Lactobacillus helveticus*, *Lactobacillus johnsonii, Lactobacillus reuteri, Lactobacillus rhamnosus (Lactobacillus* GG), *Lactobacillus sake, Lactococcus lactis, Micrococcus varians, Pediococcus acidilactici, Pediococcus pentosaceus, Pediococcus acidilactici, Pediococcus halophilus, Streptococcus faecalis, Streptococcus thermophilus, Staphylococcus carnosus,* and *Staphylococcus xylosus.*

The probiotic micro-organisms may be in powdered, dried form, especially in spore form for micro-organisms which form spores. Further, if desired, the probiotic micro-organism may be encapsulated to additionally increase the feasibility of survival, for example in a sugar matrix, fat matrix or polysaccharide matrix. If a probiotic micro-organism is used, the nutritional composition preferably contains about 10⁴ to about 10¹⁰ cells of the probiotic micro-organism per gram of the nutritional composition, more preferably about 10⁶ to about 10⁸ cells of the probiotic micro-organism per gram. The nutritional composition may contain about 0.5 wt.-% to about 20 wt.-% of the mixture of the probiotic micro-organism, preferably about 1 wt.-% to about 6 wt.-%, for example about 3 wt.-% to about 6 wt.-%, each on the basis of the dry weight of the total composition.

One or more food grade emulsifiers may be incorporated into the nutritional composition if desired, for example diacetyl tartaric acid esters of mono- and di-glycerides, lecithin and mono- and di-glycerides. Similarly, suitable salts and stabilizers may be included.

The nutritional composition may be used for humans and/or mammals, in particular pets, such as cats and dogs, and will be adapted to the specific nutritional requirements of the respective target mammal.

The said composition may be administered to the respective individual as a supplement to the normal diet or as a component of a nutritionally complete food. The food composition may also be a complete and nutritionally balanced food.

In one embodiment, a nutritionally complete food or pet food can be prepared. The nutritionally complete food or pet food may be in any suitable form, for example in dried form, semi-moist form or wet form, it may be a chilled or shelf stable pet food product. These foods or pet foods may be produced as is conventional. Apart from the nutritional composition according to the present invention, these foods or pet foods may include any one or more of a carbohydrate source, a protein source and lipid source.

For dried foods or dried pet foods a suitable process is extrusion cooking, although baking and other suitable processes may be used. When pet food is extrusion cooked, the dried pet food is usually provided in the form of a kibble. If a prebiotic is used, the prebiotic may be admixed with the other ingredients of the dried food or pet food prior to processing. A suitable process is described e.g. in EP-A-0850569. If a probiotic micro-organism is used, the organism is best coated onto or filled into the dried pet food. A suitable process is described in EP-A-0862863.

For wet foods or wet pet foods, the processes described in US-4,781,939 and US-5,132,137 may be used to produce simulated meat products. Other procedures for producing chunk type products may also be used, for example cooking in a steam oven. Alternatively, loaf type products may be produced by emulsifying a suitable meat material to produce a meat emulsion, adding a suitable gelling agent, and heating the meat emulsion prior to filling into cans or other containers.

Nutritional compositions for human consumption may be for example a nutritional complete formula, a dairy product, a chilled or shelf stable beverage, soup, a dietary supplement, a meal replacement, and a nutritional bar or a confectionery.

The nutritional composition can be enterally administrable, for example in the form of a powder, a liquid concentrate, or a ready-to-drink beverage. If it is desired to produce a powdered nutritional formula, the homogenized mixture is transferred to a suitable drying apparatus such as a spray drier or freeze drier and converted to powder.

In addition thereto, a usual food product, such as e.g. a fermented milk, a yogurt, a fresh cheese, a renneted milk, a confectionery bar, breakfast cereal flakes or bars, drinks, milk powders, soy-based products, non-milk fermented products or nutritional supplements for clinical nutrition may also be enriched with a nutritional composition according to the present invention.

Preferably, the amount of the food composition to be consumed by the human and/or mammal to obtain a beneficial effect will depend upon its size, its type, and its age and may be chosen according to the general knowledge in the art by a skilled person.

The nutritional composition according to the present invention may be expected too be highly accepted by the consumers as it provides its beneficial effects on the skin without requiring a restriction of the fat content of the diet. Thus, the present invention overcomes a prejudice present in the art, as previously it has been supposed that such effects may only achieved only on basis of a diet relatively low in fat.

It should be appreciated that the components of a nutritional composition according to present invention provide therein a synergetic activity exceeding and/or different from the activity of the respective component when used alone.

The following examples are given by way of illustration only and in should not be construed as limiting the subject matter of the present application. All percentages are given by weight unless otherwise indicated. They are preceded by a brief description of the figure.

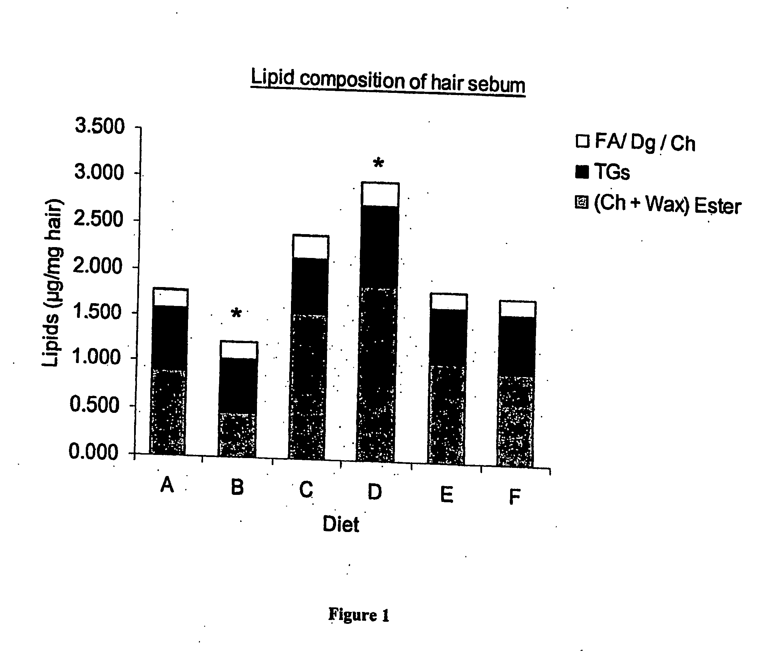

**Figure 1** is a diagram showing the hair sebum lipid amounts measured by HPLC, on male mice C57BL/6J fed diets A to E as described in example 1 and a diet F. Sebum lipids were grouped in 3 classes: Cholesterol + diglycerides and triglycerides and cholesterol and wax esters.

### Example 1

### Observations in a murine model

### Study design and diets

Male mice C57BL/6J were obtained from Iffa credo (France) at 9 weeks of age. Upon arrival mice were housed by groups of 6 animals. After 3 weeks adaptation, mice (12 weeks old) were randomized in 5 groups (A to E) of 12 mice each and housed individually. Mice had free access to water and were submitted to 12 hours light and dark cycles.

Dietary intervention in adult mice was a 3 months feeding from 12 to 15 months of age.

Dietary intervention in old mice was a feeding of 21 to 24 months or a long-term feeding of over 21 months (from 3 to 24 months of age).

All animal groups were fed Ad lib (ad libidum) except for the group of caloric restricted mice which was fed 67 % of the daily food consumed by the control Ad lib group. Animal weight was measured once a week.

The control diet (diet A) composed of 18% proteins (soy and whey), 11% fat (soybean oil), 59% carbohydrates (starch + sucrose) and 10% cellulose was supplemented with either a cocktail of antioxidants comprising vitamin C, vitamin E, grape seed extract and cysteine (diet C) and/or L-carnitine (diet D and E respectively). For caloric restriction (diet B) fat, starch and sucrose were reduced to provide 67% of the daily calorie consumption of the Adlib control group while providing 100% for proteins, minerals and vitamins. These diets are as follows:
**Diet A - Control:** 18% proteins (soy and whey), 11% fat, 59% carbohydrates, 5% cellulose
**Diet AL - Control:** long term administration of Diet A over 24 months;
**Diet B - Caloric restriction:** 18% proteins (soy and whey), 7.7% fat, 32.5% carbohydrates, 5% cellulose;
**Diet BL - Control:** long term administration of Diet B over 24 months;
**Diet C - Cocktail of antioxidants:** Diet A + 0.19% vitamin C, 0.03% vitamin E, 0.075% grape seed extract, 0.4% cysteine;
**Diet D:** Diet A + 0.3% L- carnitine + cocktail of antioxidants of diet C;
**Diet DL - control:** long term administration of Diet D over 24 months;
**Diet E :** Diet A + 0.3% L- carnitine;
**Diet F:** Diet A + 0.3% L-carnitine.

As mentioned above, the study as described in the present invention was inter alia originally directed to determine the effects of caloric restrictions on the mortality of mice, for which purposes Diet B was administered to the animals.

It was noted that one of the comparative diets - Diet D - had a protective activity with respect to inflammatory skin diseases and helped to avoid dermatitis, in particular ulcerative dermatitis, and increases the lipid secretion in sebum.

### Ulcerative dermatitis in C57BL/6J

Overgrooming, alopecia and dermatitis are characteristics of C57BL/6J mice, inbred and can occur as early as 2-4 months of age. Table 1, as shown below shows the incidence of dermatitis in the various diet groups.

**Table 1**

| Group | Number of cases | group size | Incidence | Period |
|---|---|---|---|---|
| A | 2 | 20 | 0.10 | 21-24 months |
| B | 2 | 19 | 0.11 | 21-24 months |
| C | 3 | 19 | 0.16 | 21-24 months |
| D | 0 | 19 | 0.00 | 21-24 months |
| E | 3 | 19 | 0.16 | 21-24 months |
| AL | 10 | 30 | 0.33 | over 24 months |
| DL | 3 | 30 | 0.10 | over 24 months |
| BL | 2 | 24 | 0.08 | over 24 months |

The mice which received a diet comprising a composition according to the present invention (Diet D) had a significant lower incidence of dermatitis as in the other diet groups.

Many of the mice belonging to the control group (AL group) developed at mid-life (12 to 18 months of age) ulcerative dermatitis that lead to extensive skin damage, skin infections and important weight loss. In particular, mice on control diet experienced a high incidence of mortality due to skin disorders.

Mice fed the control diet were more prone to skin disorders than the mice belonging to the other diet groups. Few skin lesions were observed in groups DL and BL at the end of the study (2 for BL, 2 for DL). Thus, a diet with caloric restriction (B, BL) and diet D (DL, D) appeared to have a protective effect

Additionally, it has been noticed in the present study that mice of control group A, developed dermatitis by 12 months of age, i.e. later than reported previously by the Jackson Laboratory (USA) Thus, the control diet itself seem to provide a protective factor, and further studies indicated that this protective factor is the relatively high content of alpha-linolenic acid in the soybean oil which has been administered during the study. Table 2 shows the content of alpha-linolenic acid and linoleic acid of soybean oil compared to corn oil.

**Table 2**

| **Essential fatty acids (FA) present in the diet (% of total FA)** | | |
|---|---|---|
| | linoleic acid (LA) 18:2 n-6 | alpha-linolenic acid (ALA) 18:3 n-3 |
| Corn oil | 57 | 0.9 |
| Soybean oil | 53 | 7 |

Results superior to the standard diets with respect to a stimulation of the lipid metabolism were also obtained when using a diet comprising L-carnitine and two of the following antioxidants: vitamin C, vitamin E, grape seed extract and cysteine.

### Example 2 : Measurement of hair sebum lipids in adult and old mice

Hair sebum lipids of mice C57BL/6J of example 1, fed with the diets A, B, C, D and E as described in example 1, were measured by HPLC. Sebum lipids were grouped in 3 classes: (1) polar lipids: diglycerides, fatty acid and cholesterol (2) triglycerides and (3) non-polar lipids: cholesterol and wax esters.

### Measurements of hair sebum lipids in Adult mice (15 months old)

The results that are represented in Tables 3 and 4show that hair sebum total lipids amount, non-polar lipids (cholesterol and wax esters) and polar lipids (diglycerides fatty acid and cholesterol) were increased in adult mice fed diet D (Table 3, grey box). The lipid balance however was not significantly modified in diet D (Table 4, light box). On the opposite, mice fed caloric restriction had significantly reduced amount of total lipids as well as non-polar lipids and triglycerides in the sebum (Table 3, grey box). In addition, lipid balance was modified in the caloric restricted mice (Table 4, light box) with a decrease proportion of triglycerides and an increase proportion of polar lipids.

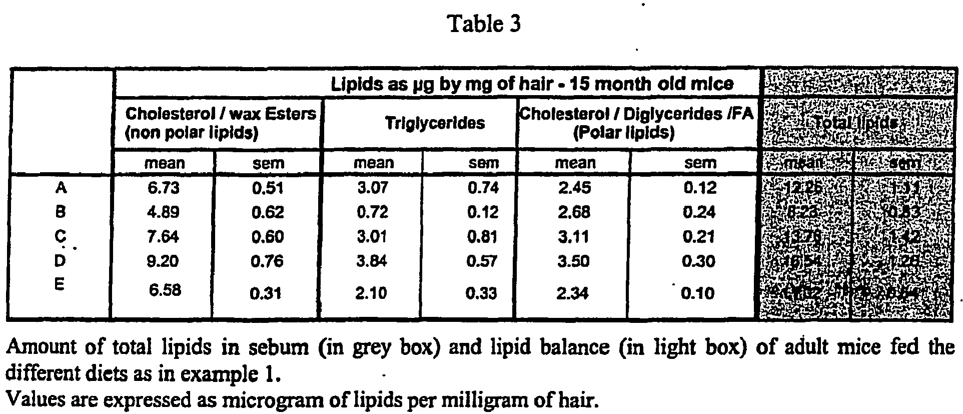

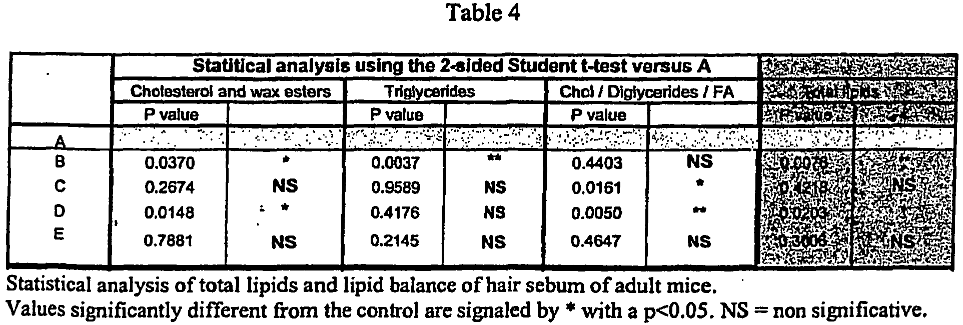

### Measurements of hair sebum lipid in old (24 months) mice:

Tables 5 and 6 represent the results of the dietary supplementation as stated in example 1 for old mice. Total lipid hair sebum amount from mice fed diets C, D and E were increased significantly contrarily to the caloric restricted mice, which show a decrease of total hair sebum lipid. Lipid balance was modified in diets B, BL, C and D in a similar manner, with an increased proportion of cholesterol and wax esters and a decrease proportion of cholesterol, diglycerides and fatty acid lipid classes. In addition caloric restriction short-term shows a decrease in triglycerides as seen in adult mice.

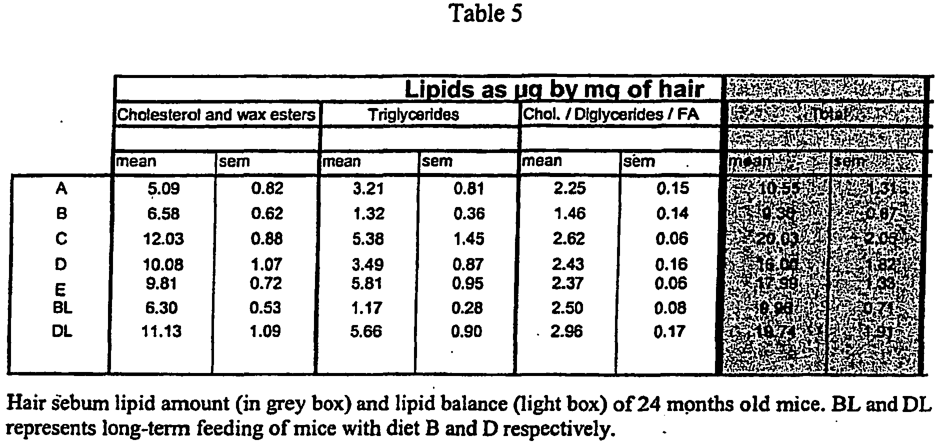

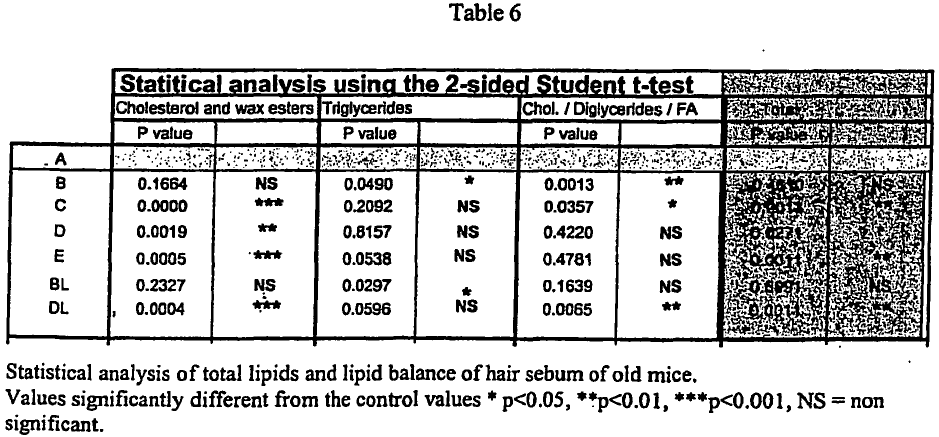

## Claims

1. An ingestable composition comprising L-carnitine and a least a component having an anti-oxidative activity selected from the group consisting of vitamin E; vitamin C, carotenoids; ubiquinones; tea catechins; coffee extracts containing polyphenols and/or diterpenes; ginkgo biloba extracts; grape or grape seed extracts rich in proanthocyanidins; spice extracts; soy extracts containing isoflavones, phytoestrogens; ursodeoxycholic acid; ursolic acid; ginseng and gingenosides and natural sources thereof; a source of thiols, preferably lipoic acid, cysteine, cystine, methionine, S-adenosyl-methionine, taurine, glutathione or natural source thereof; or mixture thereof for use in the prevention of dry skin condition, or sensible or reactive skin condition of an animal or a human being.

2. An ingestable composition comprising L-carnitine and a least a component having an anti-oxidative activity for the stimulation of the lipid metabolism in the skin of an animal or a human being for use in the prevention of onset or incidence of ulcers associated with diabetes, circulation disturbances, physical, chemical or microbial noxae or eczema.

3. A composition for use according to claim 1 or 2, for reducing skin itching.

4. An ingestable composition comprising L-carnitine and a least a component having an anti-oxidative activity for the stimulation of the lipid metabolism in the skin of an animal or a human being for use in the reduction of itching and for preventing a dry skin condition or sensible skin condition.

5. A composition for use according to any of claim 1 to 4 wherein the composition is a medicament.

6. A composition for use according to any of claim 1 to 4, wherein the composition is a food, a functional food, a nutritionally complete pet or human food or a dietary supplement.

7. A composition for use according to any of the preceding claims for increasing the lipid secretion in the sebum and / or for producing a protective sebum layer on the skin.

8. A composition for use according to any of the preceding claims, for treating ulcerative dermatitis or dermatitis.

9. A composition for use according to any of the preceding claims, wherein the amount of L-carnitine administrated daily is from 1 mg to 1g per kg of body weight / day, preferably of from 5 mg to 250 mg per kg of body weight / day.

10. A composition for use according to any of the preceding claims, wherein the amount of the component having an anti-oxidative activity is from 0.025 mg to 250 mg per kg of body weight / day.

11. A composition for use according to any of the preceding claims, wherein the ingestable composition or food contains a source of fat, which comprises unsaturated fatty acid or is enriched with unsaturated fatty acid.

12. A composition for use according to claim 11, wherein the unsaturated fatty acid is alpha-linolenic acid.

13. A composition for use according to any of claims 11 or 12, wherein said source of fat is selected from the group consisting of an animal fat, preferably tallow or fish oil, more preferably beef tallow, or a vegetable fat, preferably corn oil, sunflower oil, safflower oil, rape seed oil, soy bean oil, olive oil, borage oil, blackcurrant seed oil.

14. A composition for use according to any of claims 11 to 13, wherein the amount of said source of fat in the composition is at least 0.1 % by weight on basis of the total weight of the composition.

## Patentansprüche

1. Einnehmbare Zusammensetzung mit L-Carnitin und mindestens einer Komponente mit einer antioxidativen Wirkung, ausgewählt aus der Gruppe aufweisend Vitamin E; Vitamin C; Carotinoide; Ubichinone; Tee-Catechine; Kaffee-Extrakte enthaltend Polyphenole und/oder Diterpene; Gingko-Biloba-Extrakte; Trauben oder Traubenkernextrakte, reich an Proanthocyanidinen; Gewürzextrakte; Isoflavone enthaltende Sojaextrakte; Phytoöstrogene; Ursodeoxycholsäure; Ursolsäure; Ginseng und Ginsenoside und natürliche Quellen davon; eine Quelle von Thiolen, vorzugsweise Liponsäure; Cystein; Cystin; Methionin; S-Adenosyl-Methionin; Taurin; Glutathion oder natürliche Quellen davon; oder Mischungen davon zur Verwendung bei der Vorbeugung eines trockenen Hautzustandes oder sensiblen oder reaktiven Hautzustandes eines Tieres oder eines Menschen.

2. Einnehmbare Zusammensetzung mit L-Carnitin und mindestens einer Komponente mit einer antioxidativen Wirkung für die Stimulation des Lipid-Metabolismus in der Haut eines Tieres oder eines Menschen zur Verwendung in der Verhütung des Ausbruchs oder Auftretens von Geschwüren im Zusammenhang mit Diabetes, Durchblutungsstörungen, physikalischen chemischen oder mikrobiellen Noxen oder Ekzemen.

3. Zusammensetzung zur Verwendung nach Anspruch 1 oder 2 zur Reduzierung des Hautjuckreizes.

4. Einnehmbare Zusammensetzung mit L-Carnitin und mindestens einer Komponente mit einer antioxidativen Wirkung für die Stimulation des Lipid-Metabolismus in der Haut eines Tieres oder eines Menschen zur Verwendung bei der Reduzierung des Juckreizes und zur Vorbeugung eines trockenen oder sensiblen Hautzustandes.

5. Zusammensetzung zur Verwendung nach einem der Ansprüche 1 bis 4, wobei die Zusammensetzung ein Medikament ist.

6. Zusammensetzung zur Verwendung nach einem der Ansprüche 1 bis 4, wobei die Zusammensetzung ein Lebensmittel, ein funktionelle Lebensmittel, ein vollwertiges Haustierfutter oder ein Lebensmittel für den Menschen oder eine Nahrungsergänzung ist.

7. Zusammensetzung zur Verwendung nach einem der vorhergehenden Ansprüche zur Erhöhung der Lipidsekretion in dem Sebum und/oder zur Herstellung einer Sebumschutzschicht auf der Haut.

8. Zusammensetzung zur Verwendung nach einem der vorhergehenden Ansprüche zur Behandlung ulzerativer Dermatitis oder Dermatitis.

9. Zusammensetzung zur Verwendung nach einem der vorhergehenden Ansprüche, wobei die Menge an täglich verabreichtem L-Carnitin von 1 mg bis 1 g pro kg Körpergewicht/Tag beträgt, vorzugsweise von 5 mg bis 250 mg pro kg Körpergewicht/Tag.

10. Zusammensetzung zur Verwendung nach einem der vorhergehenden Ansprüche, wobei die Menge der Komponente mit einer antioxidativen Wirkung von 0,025 mg bis 250 mg pro kg Körpergewicht/Tag beträgt.

11. Zusammensetzung zur Verwendung nach einem der vorhergehenden Ansprüche, wobei die einnehmbare Zusammensetzung oder das Lebensmittel eine Fettquelle aufweist, die eine ungesättigte Fettsäure umfasst, oder mit einer ungesättigten Fettsäure angereichert ist.

12. Zusammensetzung zur Verwendung nach Anspruch 11, wobei die ungesättigte Fettsäure alpha-Linolensäure ist.

13. Zusammensetzung zur Verwendung nach Anspruch 11 oder 12, wobei die Fettquelle aus der Gruppe ausgewählt ist aufweisend ein tierisches Fett, vorzugsweise Talg oder Fischöl, weiter bevorzugt Rindertalg, oder ein Pflanzenfett, vorzugsweise Maisöl, Sonnenblumenöl, Safloröl, Rapsöl, Sojaöl, Olivenöl, Borretschöl, schwarzes Johannisbeerkernöl.

14. Zusammensetzung zur Verwendung nach einem der Ansprüche 11 bis 13, wobei die Menge der Fettquelle in der Zusammensetzung mindestens 0,1 Gew.-% auf der Basis des Gesamtgewichts der Zusammensetzung beträgt.

## Revendications

1. Composition ingérable comprenant de la L-carnitine et au moins un composant présentant une activité anti-oxydante choisi parmi le groupe constitué de vitamine E ; vitamine C ; caroténoïdes ; ubiquinones ; catéchines de thé ; extraits de café contenant des polyphénols et/ou diterpènes ; extraits de ginkgo biloba ; raisin ou extraits de pépin de raisin riches en proanthocyanidines ; extraits d'épice ; extraits de soja contenant des isoflavones, phytoestrogènes ; acide ursodésoxycholique ; acide ursolique ; ginseng et ginsenosides et leurs sources naturelles ; une source de thiols, de préférence l'acide lipoïque, la cystéine, la cystine, la méthionine, la S-adénosyl-méthionine, la taurine, le glutathion ou leur source naturelle ; ou leurs mélanges pour une utilisation dans la prévention d'un état de peau sèche ou d'un état de peau sensible ou réactive chez un animal ou un être humain.

2. Composition ingérable comprenant de la L-carnitine et au moins un composant présentant une activité anti-oxydante pour la stimulation du métabolisme lipidique dans la peau d'un animal ou d'un être humain pour une utilisation dans la prévention du début ou de la survenue d'ulcères associés à du diabète, des troubles de la circulation, de nuisances physiques, chimiques ou microbiennes ou de l'eczéma.

3. Composition pour une utilisation selon la revendication 1 ou 2, pour réduire des démangeaisons cutanées.

4. Composition ingérable comprenant de la L-carnitine et au moins un composant présentant une activité anti-oxydante pour la stimulation du métabolisme lipidique dans la peau d'un animal ou d'un être humain pour une utilisation dans la réduction de démangeaisons ou pour la prévention d'un état de peau sèche ou d'un état de peau sensible.

5. Composition pour une utilisation selon l'une quelconque des revendications 1 à 4, dans laquelle la composition est un médicament.

6. Composition pour une utilisation selon l'une quelconque des revendications 1 à 4, dans laquelle la composition est un aliment, un aliment fonctionnel, un aliment pour animal de compagnie ou être humain nutritionnellement complet ou un supplément diététique.

7. Composition pour une utilisation selon l'une quelconque des revendications précédentes pour augmenter la sécrétion de lipides dans le sébum et/ou pour produire une couche protectrice de sébum sur la peau.

8. Composition pour une utilisation selon l'une quelconque des revendications précédentes, pour le traitement de la dermatite ulcéreuse ou de la dermatite.

9. Composition pour une utilisation selon l'une quelconque des revendications précédentes, dans laquelle la quantité de L-carnitine administrée quotidiennement est de 1 mg à 1 g par kg de poids corporel / jour, de préférence de 5 mg à 250 mg par kg de poids corporel / jour.

10. Composition pour une utilisation selon l'une quelconque des revendications précédentes, dans laquelle la quantité du composant présentant une activité anti-oxydante est de 0,025 mg à 250 mg par kg de poids corporel / jour.

11. Composition pour une utilisation selon l'une quelconque des revendications précédentes, dans laquelle la composition ingérable ou l'aliment contient une source de matière grasse, qui comprend de l'acide gras insaturé ou qui est enrichi en acide gras insaturé.

12. Composition pour une utilisation selon la revendication 11, dans laquelle l'acide gras insaturé est l'acide alpha-linolénique.

13. Composition pour une utilisation selon l'une quelconque des revendications 11 ou 12, dans laquelle ladite source de matière grasse est choisie parmi le groupe constitué d'une matière grasse animale, de préférence le suif ou l'huile de poisson, plus préférablement le suif de boeuf, ou d'une matière grasse végétale, de préférence l'huile de maïs, l'huile de tournesol, l'huile de carthame, l'huile de colza, l'huile de soja, l'huile d'olive, l'huile de bourrache, l'huile de pépin de cassis.

14. Composition pour une utilisation selon l'une quelconque des revendications 11 à 13, dans laquelle la quantité de ladite source de matière grasse dans la composition est d'au moins 0,1 % en poids sur la base du poids total de la composition.
